# EUROPÄISCHE PATENTANMELDUNG

(11) **EP 4 553 211 A1**
(43) Veröffentlichungstag der Anmeldung: **14.05.2025**
(21) Anmeldenummer: 24198893.0
(22) Anmeldetag: 06.09.2024
(51) Int. Cl.: D04H 1/492, D04H 1/498, D04H 3/11, A61K 8/02

(54) **VERFAHREN ZUR HERSTELLUNG VON MIT TRÄNKUNGSMITTEL GETRÄNKTEN TUCHPRODUKTEN**

(30) Priorität: 09.11.2023 DE 102023131186
(71) Anmelder: Dr. Schumacher GmbH, 34323 Malsfeld-Beiseförth (DE)
(72) Erfinder: Siwek, Wojciech, 59-800 Lubán (PL); Birkelbach, Anke, 34323 Malsfeld (DE); Heidari, Mina, 34323 Malsfeld (DE)
(74) Vertreter: Sandvoß, Stefanie

(57) **Zusammenfassung**

Die Erfindung betrifft ein Verfahren zur Herstellung von mit Tränkungsmittel getränkten Tuchprodukten, wobei das Verfahren die Verfestigung und/oder Verbindung von Fasern und/oder Vliesstoffen mittels Wasserstrahlverfestigung beinhaltet, wobei nach der Wasserstrahlverfestigung ein Konzentrat eines Tränkungsmittels auf die noch mit Wasser getränkten miteinander verbundenen und/oder verfestigten Fasern und/oder Vliesstoffe aufgebracht wird, wobei das Konzentrat zusammen mit Wasser, mit dem die miteinander verbundenen und/oder verfestigten Fasern und/oder Vliesstoffe nach der Wasserstrahlverfestigung getränkt sind, das Tränkungsmittel bildet, mit dem die getränkten Tuchprodukte getränkt sind.

## Beschreibung

Die vorliegende Erfindung betrifft ein neues Verfahren zur Herstellung von mit Tränkungsmittel getränkten Tuchprodukten unter Verfestigung und/oder Verbindung von Fasern oder Vliesstoffen im Rahmen der Herstellung von Tuchprodukten.

Tuchprodukte finden in Abhängigkeit ihrer Beschaffenheit sehr vielfältigen Einsatz, insbesondere im kosmetischen und hygienischen Bereich sowie auch für zahlreiche Reinigungsanwendungen. Je nach gewünschter Anwendung, die oftmals nass bzw. feucht erfolgt, können die Tuchprodukte bereits mit einem Tränkungsmittel imprägniert und einzeln oder zu Einheiten mit einer vorbestimmten Anzahl an Tuchprodukten verpackt sein, oder sie können auch im ungetränkten Zustand verpackt sein und ggf. erst nach der Entnahme aus der Verpackung befeuchtet oder getränkt werden, sofern dies gewünscht oder erforderlich ist.

Je nach gewünschter Anwendung der Tuchprodukte umfassen diese mehrere Schichten Vliesstoff, die gleich oder unterschiedlich zusammengesetzt sein können. Im Fall von Einweg-Wischtüchern zur Reinigung von Oberflächen ist es bekannt, dass diese mehrere Schichten Vliesstoff umfassen sollten, die in der Regel unterschiedlich zusammengesetzt sind. Dies bewirkt einerseits eine hinreichende Stabilität des Wischtuchs und kann andererseits bei Schichten aus unterschiedlich zusammengesetzten Vliesstoffen bei entsprechender Wahl der Vliesstoffe gewährleisten, dass beispielsweise eine erste Schicht eine gute Flüssigkeitsaufnahme bietet, während eine zweite Schicht eine gute Flüssigkeitsabgabe bietet.

Aus dem Stand der Technik sind bereits seit langem verschiedene Verfahren zur Herstellung von Vliesstoffen aus losen Fasern und auch zur Herstellung von mehrschichtigen Tuchprodukten durch Verbindung mehrerer Lagen Vliesstoff miteinander bekannt. Bekannte Verfahren, durch die aus losen Fasern ein vorgefestigter Faserverbund erhalten werden kann, sind beispielsweise das Trockenvliesverfahren (Drylaid), das Nassvliesverfahren (Wetlaid), das Meltblown-Verfahren und das Spinnvliesverfahren. Neben den vorgenannten zur "Web formation"-Technologie gehörenden Verfahren finden auch zur "Web bonding"-Technologie gehörende Verfahren wie Kalandrierung, Wasserstrahlverfestigung (Hydroentanglement, spunlace), Vernadelung (Needle punch) sowie Air through Bonding Anwendung.

Bei der Wasserstrahlverfestigung werden lose Fasern mit unter hohem Druck aus Düsen austretenden Wasserstrahlen miteinander verwirbelt, wodurch eine Verfestigung zu einem Faserverbund erreicht wird. Aus der PCT/EP2023/072187 ist zudem bekannt, dass die Wasserstrahlverfestigung auch zur Verbindung bereits vorgefestigter Schichten geeignet ist, da durch den hohen Druck, mit dem die Wasserstrahlen bei der Wasserstrahlverfestigung auf die Vliesschichten auftreffen, selbst die bereits zu einem Faserverbund verfestigten Fasern unterschiedlicher Schichten noch eine Verbindung miteinander eingehen.

Weitere Verfahren zur Verbindung bereits vorgefestigter Schichten sind neben der Wasserstrahlverfestigung insbesondere die Ultraschallverfestigung und die Vernadelung.

Die DE 10 2021 122 041 B1 beschreibt ein mehrlagiges Einweg-Bodenwischtuch mit abrasivem Streifen, das mindestens eine Reinigungsschicht und eine Kernschicht umfasst, die beide Cellulose enthalten und vollständig aus Naturfasern und/oder Regeneratfasern bestehen, wobei auf oder in der Reinigungsschicht ein Kratzstreifen mit im Vergleich zu der restlichen Reinigungsschicht erhöhten abrasiven Eigenschaften angeordnet ist, und wobei der Kratzstreifen Hanf enthält und ebenfalls vollständig aus Naturfasern und/oder Regeneratfasern besteht. Die Reinigungsschicht und die Kernschicht sowie optionale weitere Schichten sind dabei vorzugsweise mittels Mikro-Entangling durch mittels Ultraschall erzeugter Bewegung miteinander verbunden.

Die DE 101 64 640 A1 beschreibt ein Verfahren zur Ausrüstung von fluidstrahlverfestigten Flächengebilden, bei dem zwischen der Fluidstrahlverfestigung und einem Trocknungsprozess eine Ausrüstung der textilen Flächengebilde mit einem Ausrüstungsmittel erfolgt, um beispielsweise gewünschte Oberflächeneffekte oder eine Verbesserung der Festigkeit der textilen Flächengebilde zu erreichen.

Die AT 503 625 A1 beschreibt eine Kombination eines Schmelzblasverfahrens mit einem Wasserstrahlverfestigungsschritt ohne vorherige Trocknung des Produktes.

Die DD 246 129 A1 beschreibt ein Transportelement zur Herstellung von Vliesstoffen, deren Verfestigung ohne Bindemittel mittels unter hohem Druck stehender Fluidstrahlen erfolgt.

### Aufgabe der Erfindung

Es ist die Aufgabe der vorliegenden Erfindung, ein alternatives Verfahren zur Herstellung von getränkten Tuchprodukten unter Verfestigung und/oder Verbindung von Fasern und/oder Vliesstoffen bereitzustellen, das sich insbesondere für die Herstellung von mit tensidhaltigem Tränkungsmittel getränkten Tuchprodukten eignet. Dieses alternative Herstellungsverfahren soll sich insbesondere durch eine im Vergleich zu aus dem Stand der Technik bekannten Verfahren energie- und ressourcenschonendere Verfahrensführung auszeichnen. Vorzugsweise sollen bei der Durchführung des Verfahrens darüber hinaus weniger Abfallprodukte anfallen.

### Allgemeine Beschreibung der Erfindung

Die Erfindung löst diese Aufgabe mit den Merkmalen der Ansprüche und insbesondere mit einem Verfahren zur Herstellung von mit Tränkungsmittel getränkten Tuchprodukten, das die Verfestigung und/oder Verbindung von Fasern und/oder Vliesstoffen mittels Wasserstrahlverfestigung beinhaltet, und bei dem nach der Wasserstrahlverfestigung ein Konzentrat eines Tränkungsmittels auf die noch mit Wasser getränkten miteinander verbundenen und/oder verfestigten Fasern und/oder Vliesstoffe aufgebracht wird, wobei das Konzentrat zusammen mit Wasser, mit dem die miteinander verbundenen und/oder verfestigten Fasern und/oder Vliesstoffe nach der Wasserstrahlverfestigung getränkt sind, das Tränkungsmittel bildet, mit dem die getränkten Tuchprodukte getränkt sind.

Die in dem erfindungsgemäßen Verfahren eingesetzten Fasern können gleichartige oder unterschiedliche beliebige Fasern sein, insbesondere natürliche Fasern sowie auch Kunstfasern.

Unter "Vliesstoffen" sind aus Fasern bestehende, verfestigte Stoffe zu verstehen, in denen die Fasern bereits eine feste Verbindung eingegangen sind. Die in den Vliesstoffen enthaltenen Fasern können ebenfalls gleichartige oder unterschiedliche beliebige Fasern sein, insbesondere natürliche Fasern sowie Kunstfasern. In Ausführungsformen, in denen zwei oder mehr Vliesstoffe durch das erfindungsgemäße Verfahren miteinander verbunden werden sollen, sind die Vliesstoffe in der Regel unterschiedlich zusammengesetzt, um in dem resultierenden Tuchprodukt unterschiedliche Funktionen zu erfüllen, wobei jeder der Vliesstoffe gleichartige oder unterschiedliche beliebige Fasern enthalten kann, die unabhängig voneinander natürliche Fasern und/oder Kunstfasern sein können. Hier zeigt sich bereits ein erster Vorteil des erfindungsgemäßen Verfahrens, denn aufgrund der Verbindung mittels Wasserstrahlen ist die Zusammensetzung der Vliesstoffe nicht auf Kunstfasern beschränkt, wie sie es wäre, wenn die Verbindung der Vliesstoffe auf thermischem Wege erfolgen würde.

Ein "Verbinden" von Fasern oder Vliesstoffen durch Wasserstrahlverfestigung meint im Rahmen der vorliegenden Erfindung in erster Linie ein Verwirren bzw. Verhaken der losen Fasern oder der Faserenden in einem Vliesstoff ineinander, und keine stoffschlüssige Verbindung.

In Ausführungsformen des erfindungsgemäßen Verfahrens, in denen zwei oder mehr Vliesstoffe miteinander verbunden werden, können die eingesetzten Vliesstoffe optional durch Herstellungstechnologien, die zur "Web formation"-Technologie oder zur "Web bonding"-Technologie gehören, hergestellt worden sein. Bevorzugte zur "Web formation"-Technologie gehörende Verfahren sind Kardieren, Airlaid-Verfahren, Wetlaid-Verfahren, Meltblown-Verfahren und Spunlaid-Verfahren. Bevorzugte zur "Web bonding"-Technologie gehörende Verfahren sind hingegen Kalandrierung, Wasserstrahlverfestigung, Vernadelung sowie Air through Bonding.

Im Rahmen der vorliegenden Erfindung wurde die Idee weiterentwickelt, lose Fasern oder bereits verfestigte Vliesstoffe durch Wasserstrahlverfestigung miteinander zu verfestigen bzw. zu verbinden.

Bei der bereits bekannten Verbindung von Vliesstoffen mittels Wasserstrahlverfestigung erfolgt im Stand der Technik nach dem Schritt der Wasserstrahlverfestigung jeweils ein Trocknungsschritt, um das Wasser, mit dem die Fasern nach der Wasserstrahlverfestigung getränkt sind, wieder zu entfernen. Zum Erhalt von mit Tränkungsmittel getränkten Tuchprodukten erfolgt dann erst in einem nachfolgenden dritten Schritt die Tränkung mit dem gewünschten Tränkungsmittel.

Erfindungsgemäß wird hingegen ganz oder teilweise auf den Trocknungsschritt, der im Stand der Technik auf die Wasserstrahlverfestigung folgt, verzichtet, d.h. das gesamte Wasser oder ein Teil des Wassers, mit dem die miteinander verbundenen und/oder verfestigten Fasern und/oder Vliesstoffe infolge der Wasserstrahlverfestigung getränkt sind, verbleibt dort als Teil des Tränkungsmittels und bildet zusammen mit dem Konzentrat eines Tränkungsmittels, das nach der Wasserstrahlverfestigung auf die noch mit Wasser getränkten miteinander verbundenen und/oder verfestigten Fasern und/oder Vliesstoffe aufgebracht wird, das Tränkungsmittel, mit dem die getränkten Tuchprodukte getränkt sind.

Ein kompletter oder teilweiser/partieller Verzicht auf den Trocknungsschritt bedeutet im Rahmen der vorliegenden Erfindung, dass nach der Wasserstrahlverfestigung entweder gar nicht getrocknet wird (kompletter Verzicht auf den Trocknungsschritt) oder dass das infolge der Wasserstrahlverfestigung in den miteinander verbundenen und/oder verfestigten Fasern und/oder Vliesstoffen enthaltene Wasser nur teilweise, zum Beispiel zu einem Anteil von bis zu 10%, bis zu 20%, bis zu 30%, bis zu 40% oder bis zu 50%, durch Trocknen entfernt wird (teilweiser/partieller Verzicht auf den Trocknungsschritt).

Durch den erfindungsgemäßen kompletten oder teilweisen Verzicht auf den im Stand der Technik auf die Wasserstrahlverfestigung folgenden Trocknungsschritt und das Aufbringen eines Konzentrats auf die miteinander verbundenen und/oder verfestigten Fasern und/oder Vliesstoffe, das mit dem noch in diesen enthaltenen Wasser / Restwasser das Tränkungsmittel bildet, führt das erfindungsgemäße Verfahren mit signifikant reduziertem Aufwand zu dem gleichen Produkt wie das bekannte Verfahren der Verbindung einzelner Schichten Vliesstoff durch Wasserstrahlverfestigung mit anschließender vollständiger Trocknung und Tränkung des erhaltenen Tuchprodukts.

Als weiterer Vorteil fallen bei dem erfindungsgemäßen Verfahren deutlich weniger Abfallprodukte an, die es zu entsorgen bzw. aufzubereiten gilt, als bei der bekannten Herstellung von getränkten Tuchprodukten unter Verwendung von Wasserstrahlverfestigung mit anschließender Entfernung des gesamten Wassers aus den verfestigten Fasern / Vliesstoffen, bei der das zur Verfestigung verwendete und bei der nachfolgenden Trocknung zurückgewonnene Prozesswasser vollständig wieder aufbereitet werden muss. Im Gegensatz dazu fällt bei dem erfindungsgemäßen Verfahren signifikant weniger oder sogar gar kein Prozesswasser als Abfallprodukt an, da dieses als Teil des Tränkungsmittels auf den verfestigten Fasern bzw. Vliesstoffen verbleibt.

Überdies ist es weiter vorteilhaft, dass das erfindungsgemäße Verfahren im Vergleich zu der aus dem Stand der Technik bekannten Herstellung von Tuchprodukten unter Verwendung von Wasserstrahlverfestigung eine immense Energieeinsparung erlaubt, da der Trocknungsschritt zur Entfernung des für die Verbindung der Vliesstoffe verwendeten Wassers vollständig entfällt oder zumindest verkürzt wird.

Zusammenfassend bietet das erfindungsgemäße Verfahren daher energetische, zeitliche und wirtschaftliche Vorteile und schont Ressourcen.

Generell kann das Konzentrat des Tränkungsmittel, das in dem erfindungsgemäßen Verfahren auf die noch mit Wasser getränkten miteinander verbundenen und/oder verfestigten Fasern und/oder Vliesstoffe aufgebracht wird, beliebig zusammengesetzt sein und in seiner Zusammensetzung für den jeweiligen Anwendungsfall angepasst sein. Beispielsweise kann das Konzentrat des Tränkungsmittels wasser- oder ölbasiert sein und darüber hinaus einen oder mehrere weitere Inhaltsstoffe enthalten, die aus der Gruppe ausgewählt sind, die Alkohole, Tenside, Antioxidantien, Konservierungsmittel, Verdicker sowie Inhaltsstoffe mit hautpflegenden, desinfizierenden und/oder antimikrobiellen Eigenschaften umfasst oder daraus besteht. Besonders bevorzugt enthält das Konzentrat des Tränkungsmittels zumindest ein Tensid.

Gemäß einer Ausführungsform weist das erfindungsgemäße Verfahren die folgenden Schritte auf:
a) Bereitstellen von losen Fasern oder Bereitstellen von zwei oder mehr Vliesstoffen;
b) Kontaktieren der losen Fasern oder der zwei oder mehr Vliesstoffe mit Wasserstrahlen, wobei sich die losen Fasern zu einem Faserverbund verbinden oder wobei sich die zwei oder mehr Vliesstoffe miteinander verbinden;
c) Kontaktieren der mit Wasser aus der Wasserstrahlverfestigung aus Schritt b) getränkten zu einem Faserverbund verbundenen Fasern oder der mit Wasser aus der Wasserstrahlverfestigung aus Schritt b) getränkten zwei oder mehr miteinander verbundenen Vliesstoffe mit einem Konzentrat eines Tränkungsmittels.

"Getränkt" bedeutet in diesem Zusammenhang, dass Wasser aus der Wasserstrahlverfestigung den verfestigten Fasern oder miteinander verbundenen Vliesstoffen auf beliebige Weise anhaftet.

Optional weist das erfindungsgemäße Verfahren einen Schritt des partiellen Trocknens der zu einem Faserverbund verbundenen mit Wasser aus der Wasserstrahlverfestigung aus Schritt b) getränkten Fasern oder der zwei oder mehr miteinander verbundenen mit Wasser aus der Wasserstrahlverfestigung aus Schritt b) getränkten Vliesstoffe auf, wobei der Trocknungsschritt nach der Wasserstrahlverfestigung (Schritt b) und vor dem Aufbringen des Konzentrats eines Tränkungsmittels auf die noch mit Wasser getränkten miteinander verbundenen und/oder verfestigten Fasern und/oder Vliesstoffe (Schritt c) erfolgt.

Dabei ist es bevorzugt, dass der Schritt des partiellen Trocknens der zu einem Faserverbund verbundenen und mit Wasser aus der Wasserstrahlverfestigung aus Schritt b) getränkten Fasern oder der zwei oder mehr miteinander verbundenen und mit Wasser aus der Wasserstrahlverfestigung aus Schritt b) getränkten Vliesstoffe eine Messung des Feuchtigkeitsgehalts der zu einem Faserverbund verbundenen Fasern oder der zwei oder mehr miteinander verbundenen Vliesstoffe unter Verwendung eines Feuchtigkeitsmessers einschließt.

Vorteilhafterweise wird der Schritt des partiellen Trocknens der zu einem Faserverbund verbundenen und mit Wasser aus der Wasserstrahlverfestigung aus Schritt b) getränkten Fasern oder der zwei oder mehr miteinander verbundenen und mit Wasser aus der Wasserstrahlverfestigung aus Schritt b) getränkten Vliesstoffe abgebrochen, wenn der durch den Feuchtigkeitsmesser detektierte Feuchtigkeitsgehalt einen vorgegebenen Grenzwert für die in den zu einem Faserverbund verbundenen Fasern oder in den zwei oder mehr miteinander verbundenen Vliesstoffen enthaltene Restfeuchte erreicht oder unterschreitet.

Gemäß einer bevorzugten Ausführungsform erfolgt die Verbindung von zwei oder mehr Vliesstoffen in dem erfindungsgemäßen Verfahren in einer Vorrichtung, die mindestens zwei Trommeln aufweist, in denen die zu verbindenden Vliesstoffe mit den Wasserstrahlen behandelt werden, wobei die Wasserstrahlen in der ersten Trommel vorzugsweise mit einem geringeren Druck auf die zu verbindenden Vliesstoffe treffen als in der zweiten Trommel.

Bevorzugt treten dabei die Wasserstrahlen in der ersten Trommel aus mehreren Düsenstreifen aus, wobei jeder Düsenstreifen in der ersten Trommel mehrere Düsen umfasst, wobei die Wasserstrahlen aus jeder zu demselben Düsenstreifen gehörenden Düse mit dem gleichen Druck austreten und wobei die Drücke der Wasserstrahlen, die aus zu unterschiedlichen Düsenstreifen gehörenden Düsen austreten, gleich oder unterschiedlich sein können und jeweils zwischen 10 und 80 bar liegen.

Vorzugsweise gibt es in der ersten Trommel drei Düsenstreifen, wobei der Druck, mit dem die Wasserstrahlen aus den Düsen des ersten Düsenstreifens austreten, bevorzugt bei 10 bis 30 bar liegt, wobei der Druck, mit dem die Wasserstrahlen aus den Düsen des zweiten Düsenstreifens austreten, bevorzugt bei 30 bis 60 bar liegt, und wobei der Druck, mit dem die Wasserstrahlen aus den Düsen des dritten Düsenstreifens austreten, bevorzugt bei 60 bis 80 bar liegt.

Es ist weiterhin bevorzugt, dass die Wasserstrahlen in der zweiten Trommel aus mehreren Düsenstreifen austreten, wobei jeder Düsenstreifen in der zweiten Trommel mehrere Düsen umfasst, wobei die Wasserstrahlen aus jeder zu demselben Düsenstreifen gehörenden Düse mit dem gleichen Druck austreten und wobei die Drücke der Wasserstrahlen, die aus zu unterschiedlichen Düsenstreifen gehörenden Düsen austreten, gleich oder unterschiedlich sein können und jeweils zwischen 30 und 100 bar liegen, vorzugsweise zwischen 60 und 100 bar, noch bevorzugter zwischen 90 und 100 bar.

Optional weist die zweite Trommel eine Noppenstruktur auf, insbesondere eine 2D-Noppenstruktur, um eine Prägung des resultierenden Tuchprodukts zu erzielen.

Weiter optional weist die zweite Trommel auch einen Feuchtigkeitsmesser auf, der eingerichtet ist, den den verfestigten Fasern bzw. Vliesstoffen nach der Wasserstrahlverfestigung anhaftenden Feuchtigkeitsgehalt zu messen. Die optionale Trocknung in dem erfindungsgemäßen Verfahren erfolgt daher vorzugsweise in der zweiten Trommel, wobei während der Trocknung der Feuchtigkeitsgehalt in den verfestigten Fasern oder Vliesstoffen gemessen wird. Der Schritt des Trocknens wird dabei vorzugsweise abgebrochen, wenn durch den Feuchtigkeitsmesser festgestellt wird, dass ein vorbestimmter Anteil des den verfestigten Fasern bzw. Vliesstoffen nach der Wasserstrahlverfestigung anhaftenden Wassers entfernt wurde, beispielsweise ein Anteil von bis zu 10%, bis zu 20%, bis zu 30%, bis zu 40% oder bis zu 50%. Nach diesem optionalen Schritt des partiellen Trocknens wird das Konzentrat des Tränkungsmittels auf die verfestigten Fasern bzw. Vliesstoffe aufgetragen und vermischt sich mit dem Wasser, mit dem diese nach der Wasserstrahlverfestigung noch getränkt sind bzw. das diesen nach der Wasserstrahlverfestigung noch anhaftet, zu dem Tränkungsmittel, mit dem die nach dem erfindungsgemäßen Verfahren hergestellten getränkten Tuchprodukte getränkt sind.

Schließlich weist das erfindungsgemäße Verfahren nach dem Verfestigen der Fasern bzw. Verbinden der Vliesstoffe durch das Kontaktieren mit Wasserstrahlen und dem anschließenden Aufbringen des Konzentrats an Tränkungsmittel, wodurch die getränkten Tuchprodukte erhalten werden, optional noch abschließende Schritte des Schneidens der erhaltenen Tuchprodukte und des Faltens der geschnittenen Tuchprodukte auf, was nach einer ebenso optionalen Verpackung der geschnittenen und gefalteten Tuchprodukte eine Tuch-an-Tuch-Entnahme aus der Verpackung durch den Verbraucher ermöglicht.

Die in der vorstehenden Beschreibung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Verfahren zur Herstellung von mit Tränkungsmittel getränkten Tuchprodukten, wobei das Verfahren die Verfestigung und/oder Verbindung von Fasern und/oder Vliesstoffen mittels Wasserstrahlverfestigung beinhaltet, **dadurch gekennzeichnet, dass** nach der Wasserstrahlverfestigung ein Konzentrat eines Tränkungsmittels auf die noch mit Wasser getränkten miteinander verbundenen und/oder verfestigten Fasern und/oder Vliesstoffe aufgebracht wird, wobei das Konzentrat zusammen mit Wasser, mit dem die miteinander verbundenen und/oder verfestigten Fasern und/oder Vliesstoffe nach der Wasserstrahlverfestigung getränkt sind, das Tränkungsmittel bildet, mit dem die getränkten Tuchprodukte getränkt sind.

2. Verfahren nach Anspruch 1, **dadurch gekennzeichnet, dass** das Konzentrat des Tränkungsmittels tensidhaltig ist, wasser- oder ölbasiert ist und darüber hinaus einen oder mehrere weitere Inhaltsstoffe enthält, die aus der Gruppe ausgewählt sind, die Alkohole, Antioxidantien, Konservierungsmittel, Verdicker sowie Inhaltsstoffe mit hautpflegenden, desinfizierenden und/oder antimikrobiellen Eigenschaften umfasst oder daraus besteht.

3. Verfahren nach Anspruch 1 oder 2, **dadurch gekennzeichnet, dass** dieses die folgenden Schritte umfasst:
a) Bereitstellen von losen Fasern oder Bereitstellen von zwei oder mehr Vliesstoffen;
b) Kontaktieren der losen Fasern oder der zwei oder mehr Vliesstoffe mit Wasserstrahlen, wobei sich die losen Fasern zu einem Faserverbund verbinden oder wobei sich die zwei oder mehr Vliesstoffe miteinander verbinden;
c) Kontaktieren der mit Wasser aus der Wasserstrahlverfestigung aus Schritt b) getränkten zu einem Faserverbund verbundenen Fasern oder der mit Wasser aus der Wasserstrahlverfestigung aus Schritt b) getränkten zwei oder mehr miteinander verbundenen Vliesstoffe mit einem Konzentrat eines Tränkungsmittels.

4. Verfahren nach einem der voranstehenden Ansprüche, **gekennzeichnet durch** einen Schritt des partiellen Trocknens der zu einem Faserverbund verbundenen mit Wasser aus der Wasserstrahlverfestigung aus Schritt b) getränkten Fasern oder der zwei oder mehr miteinander verbundenen mit Wasser aus der Wasserstrahlverfestigung aus Schritt b) getränkten Vliesstoffe, wobei der Trocknungsschritt nach der Wasserstrahlverfestigung und vor dem Aufbringen des Konzentrats eines Tränkungsmittels auf die noch mit Wasser getränkten miteinander verbundenen und/oder verfestigten Fasern und/oder Vliesstoffe erfolgt.

5. Verfahren nach Anspruch 4, **dadurch gekennzeichnet, dass** der Schritt des partiellen Trocknens der zu einem Faserverbund verbundenen mit Wasser aus der Wasserstrahlverfestigung aus Schritt b) getränkten Fasern oder der zwei oder mehr miteinander verbundenen mit Wasser aus der Wasserstrahlverfestigung aus Schritt b) getränkten Vliesstoffe eine Messung des Feuchtigkeitsgehalts der zu einem Faserverbund verbundenen Fasern oder der zwei oder mehr miteinander verbundenen Vliesstoffe unter Verwendung eines Feuchtigkeitsmessers einschließt.

6. Verfahren nach Anspruch 5, **dadurch gekennzeichnet, dass** der Schritt des partiellen Trocknens der zu einem Faserverbund verbundenen mit Wasser aus der Wasserstrahlverfestigung aus Schritt b) getränkten Fasern oder der zwei oder mehr miteinander verbundenen mit Wasser aus der Wasserstrahlverfestigung aus Schritt b) getränkten Vliesstoffe abgebrochen wird, wenn der durch den Feuchtigkeitsmesser detektierte Feuchtigkeitsgehalt einen vorgegebenen Grenzwert für die in den zu einem Faserverbund verbundenen Fasern oder in den zwei oder mehr miteinander verbundenen Vliesstoffen enthaltene Restfeuchte erreicht.

7. Verfahren nach einem der voranstehenden Ansprüche, **dadurch gekennzeichnet, dass** die Wasserstrahlen aus einer oder mehreren Düsen austreten, bevor sie auf die Fasern oder auf die zwei oder mehr Vliesstoffe treffen.

8. Verfahren nach einem der voranstehenden Ansprüche, **dadurch gekennzeichnet, dass** die Verbindung der Fasern oder der Vliesstoffe in einer Vorrichtung erfolgt, die mindestens zwei Trommeln aufweist, in denen die zu verbindenden Fasern oder Vliesstoffe mit den Wasserstrahlen behandelt werden, wobei die Wasserstrahlen in der ersten Trommel mit einem geringeren Druck auf die zu verbindenden Fasern oder Vliesstoffe treffen als in der zweiten Trommel.

9. Verfahren nach Anspruch 8, **dadurch gekennzeichnet, dass** die Wasserstrahlen in der ersten Trommel aus mehreren Düsenstreifen austreten, wobei jeder Düsenstreifen in der ersten Trommel mehrere Düsen aufweist, wobei die Wasserstrahlen aus jeder zu demselben Düsenstreifen gehörenden Düse mit dem gleichen Druck austreten und wobei die Drücke der Wasserstrahlen, die aus zu unterschiedlichen Düsenstreifen gehörenden Düsen austreten, gleich oder unterschiedlich sein können und jeweils zwischen 10 und 80 bar liegen.

10. Verfahren nach einem der voranstehenden Ansprüche, **dadurch gekennzeichnet, dass** die Wasserstrahlen in der zweiten Trommel aus mehreren Düsenstreifen austreten, wobei jeder Düsenstreifen in der zweiten Trommel mehrere Düsen aufweist, wobei die Wasserstrahlen aus jeder zu demselben Düsenstreifen gehörenden Düse mit dem gleichen Druck austreten und wobei die Drücke der Wasserstrahlen, die aus zu unterschiedlichen Düsenstreifen gehörenden Düsen austreten, gleich oder unterschiedlich sein können und jeweils zwischen 30 und 100 bar liegen.

11. Verfahren nach einem der voranstehenden Ansprüche, **dadurch gekennzeichnet, dass** die zweite Trommel oberflächenvergrößernde Elemente auf ihrer inneren Oberfläche aufweist, wodurch eine Prägung der miteinander verbundenen Fasern oder Vliesstoffe erfolgt.

12. Verfahren nach einem der voranstehenden Ansprüche, **dadurch gekennzeichnet, dass** in der zweiten Trommel der Feuchtigkeitsgehalt der durch die Wasserstrahlverfestigung miteinander verbundenen Fasern oder Vliesstoffe bestimmt wird.

13. Verfahren nach einem der voranstehenden Ansprüche, **dadurch gekennzeichnet, dass** der Schritt des partiellen Trocknens der zu einem Faserverbund verbundenen mit Wasser aus der Wasserstrahlverfestigung aus Schritt b) getränkten Fasern oder der zwei oder mehr miteinander verbundenen mit Wasser aus der Wasserstrahlverfestigung aus Schritt b) getränkten Vliesstoffe in der zweiten Trommel erfolgt, wobei während der Trocknung der Feuchtigkeitsgehalt in den Fasern oder Vliesstoffen gemessen wird.

14. Verfahren nach Anspruch 13, wobei der Schritt des partiellen Trocknens abgebrochen wird, wenn durch einen Feuchtigkeitsmesser festgestellt wird, dass ein vorbestimmter Anteil des den verfestigten Fasern bzw. Vliesstoffen nach der Wasserstrahlverfestigung anhaftenden Wassers entfernt wurde.

15. Verfahren nach Anspruch 14, **dadurch gekennzeichnet, dass** der Schritt des partiellen Trocknens abgebrochen wird, wenn festgestellt wird, dass ein Anteil von bis zu 10%, bis zu 20%, bis zu 30%, bis zu 40% oder bis zu 50% des den verfestigten Fasern bzw. Vliesstoffen nach der Wasserstrahlverfestigung anhaftenden Wassers entfernt wurde.
